# EUROPEAN PATENT APPLICATION

(11) **EP 3 944 856 A1**
(43) Date of publication of application: **02.02.2022**
(21) Application number: 20188735.3
(22) Date of filing: 30.07.2020
(51) Int. Cl.: A61K 8/49, A61Q 5/00

(54) **USE OF PIROCTONE OLAMINE**

(71) Applicant: Unilever Global IP Ltd, Wirral, CH62 4ZD (GB)
(72) Inventor: BHOGAL Ranjit Kaur, Bedfordshire, MK44 1LQ (GB); GUNN, David Adrew, Bedfordshire, MK44 1LQ (GB); SAWICKA, Magdalena, Bedfordshire, MK44 1LQ (GB)
(74) Representative: James, Helen Sarah

(57) **Abstract**

The invention provides the cosmetic use of piroctone olamine as an active ingredient for upregulating the expression of hair fibre structural proteins in cells of the pre-emergent hair fibre; and thereby improving the structural properties of the emergent hair fibre.

## Description

### Field of the Invention

The invention relates to the use of piroctone olamine as an agent for improving structural properties of the hair fibre.

### Background of the Invention

The human hair fibre originates in the hair follicle approximately 4mm deep in the scalp skin. The formative hair fibre exists for approximately two weeks within the scalp skin while it is hardening and maturing, prior to emerging at the scalp surface. The part of the hair fibre existing below the scalp surface is termed the "pre-emergent" hair fibre.

As the hair fibre just begins to emerge from the scalp surface and for approximately 8 weeks thereafter, the hair fibre is termed "emergent." This allows for the differentiation of specific regions of the hair fibre, relative to the surface of the scalp.

The hair fibre that continues to grow past the 8 week period is then considered "post-emergent."

The emergent hair fibre grows approximately 1cm per month, and is produced by proliferation and differentiation of cells in the matrix region of the follicle, where distinct cell lineages give rise to cylindric structures of the hair fibre: the medulla, cortex, and cuticle. The cuticle, forming the thin outer sheath of the fibre is mainly responsible for hair appearance and qualities such as hair smoothness and shine, whereas mechanical properties are attributed to the inner hair cortex.

Migration of differentiating cells up the follicle is accompanied by gradual expression and deposition of specific hair keratins and keratin-associated proteins (KAPs) and simultaneous breakdown of non-keratin cell compartments. Hair keratins are the structural proteins which form keratin intermediate filaments (KIFs), the scaffolding of the hair shaft, whereas KAPs are constituents of the matrix responsible for cross-linking KIFs together.

The synthesis of hair keratins and KAPs and their correct deposition in the structures of the newly forming emergent hair fibre is essential for growing strong and healthy hair fibres, and any perturbations in this process may affect the quality of the produced fibres.
Good hair fibre quality is desired by all, and is traditionally approached by coating the hair surface with oily or film-forming cosmetic agents. Such agents may, for example, lubricate hair fibres to improve texture and alignment or fill in cuticular imperfections to improve shine and feel.

Although the above agents are effective to repair or restore post-emergent hair fibres, they do not address the early stage processes which are key to the formation and emergence of good quality hair fibres from the outset.

The present invention addresses this problem.

WO2016/191518 describes a method of improving the health of hair emerging from a scalp. However, in this case the effects are mediated by reducing local oxidative stress in the scalp milieu surrounding the formative hair. "Oxidative stress" in this context is defined by measuring the levels of (±)-9-hydroxy-10E, 12Z-octadecadienoic acid and (±)-13-hydroxy-10E, 12Z-octadecadienoic acid ("HODE") in scalp tape strips and hair samples of test subjects.

### Summary of the Invention

The invention provides the cosmetic use of piroctone olamine as an active ingredient for upregulating the expression of genes encoding hair fibre structural proteins in cells of the pre-emergent hair fibre; and thereby improving the structural properties of the emergent hair fibre.

The invention also provides piroctone olamine for use as an active ingredient for upregulating the expression of genes encoding hair fibre structural proteins in cells of the pre-emergent hair fibre and thereby improving the structural properties of the emergent hair fibre.

The invention also provides the use of piroctone olamine as an active ingredient in, and for the manufacture of, topical compositions for upregulating the expression of genes encoding hair fibre structural proteins in cells of the pre-emergent hair fibre; and thereby improving the structural properties of the emergent hair fibre.

### Detailed Description and Preferred Embodiments

### Piroctone olamine

Piroctone olamine (1-hydroxy-4-methyl-6-(2,4,4-trimethyl)-2-(1 H)pyridinone,2-aminoethanol salt) is an ethanolamine salt of the hydroxamic acid derivative piroctone. Piroctone olamine (also known as piroctone ethanolamine; is the ethanolamine salt of the hydroxamic acid derivative piroctone. It is known as an antimycotic agent (brand name Octopirox^{®}), and is used in antidandruff shampoos for the purpose of controlling the growth of *Malassezia spp.* It is believed to inhibit energy metabolism in mitochondria of pathogenic fungi by penetrating the fungal cell membrane and forming complexes with iron. The effect of piroctone olamine on the expression of genes encoding hair fibre structural proteins in cells of the pre-emergent hair fibre has not been disclosed.

### Hair fibre structural proteins

Structural properties of the emergent hair fibre include mechanical attributes such as integrity and strength, as well as surface attributes such as texture, alignment, smoothness and shine.

The term "hair fibre structural proteins" in the context of this invention denotes hair keratins and keratin-associated proteins.

Type I hair keratins are acidic and have molecular masses ranging from 40 to 48 kD, and type II hair keratins are basic to neutral and have molecular masses ranging from 58 to 65 kD. As with all intermediate filament subunit proteins, the hair keratins have a common secondary structure that consists of an N-terminal domain; 4 central alpha-helical coiled-coil domains, denoted 1A, 1B, 2A, and 2B; and a C-terminal domain. The non-alpha-helical domains of hair keratins have a high content of cysteine and proline residues, the former reflecting the use of disulfide bonding to produce a tougher, more durable structure. Keratins are obligate heteropolymers, with distinct pairs of type I and type II proteins associating to form heterodimers; these further polymerize to produce the final 10-nm keratin intermediate filament (KIF). Hair keratin genes are differentially expressed in the cuticle and cortex of the hair follicle.

Keratin-associated proteins (KAPs) are relatively small, hydrophobic proteins possessing either high cysteine or high glycine-tyrosine content, and often have characteristic repeat structures. KAPs form the matrix between the hair KIF bundles through extensive disulfide bond cross-linking with cysteine residues in the head and tail domains of hair keratins. More than 80 individual KAP members are expressed differentially in the hair cortex and cuticle during hair fibre formation.

Examples of hair fibre structural proteins encoded by genes whose expression can be upregulated by piroctone olamine in accordance with this invention are:

### Inner root sheath keratins

Inner root sheath (IRS) keratins are believed to be important for hair fibre texture and alignment. For example, disorders involving IRS keratin genes such as KRT25, 71 and 74 have been shown to lead to impaired formation of KIFs and manifest in changes in hair texture such as woolly hair phenotype.

Accordingly, examples of improved structural properties which may be imparted to the emergent hair fibre in accordance with this invention include improved hair fibre texture and alignment (as a consequence of the upregulation of genes encoding IRS keratins).

Specific examples of IRS keratins encoded by genes whose expression can be upregulated by piroctone olamine in accordance with this invention are:
Type I (acidic) inner root sheath keratins, particularly K25-28 which are encoded by KRT25-28 respectively. K25-28 are expressed in the soft-keratinizing suprabasal cells of the inner root sheath in the hair follicle. K25,K27 and K28 are expressed in the epithelial cells of all three compartments of the inner root sheath of the hair follicle (i.e. root sheath cuticle, Henle layer and Huxley layer) whereas expression of K26 is restricted to the cells forming the root sheath cuticle.
Type II (basic) inner root sheath keratins, particularly K71-75 which are encoded by KRT71-75 respectively. K71-75 are expressed in the soft-keratinizing and -cornifying cells forming the inner root sheath of the hair follicle. K71 is expressed in all three compartments of the inner root sheath of the hair follicle and can form heterodimers with each of the three acidic K25, K27 and K28, which are also specific tor the inner root sheath of the hair follicle. K72 is expressed late in the keratinizing cells of the cuticle of the inner root sheath. K73 is produced in the suprabasal cells of the root sheath cuticle. K74 is expressed exclusively in the keratinizing epithelial cells of the Huxley layer of the inner root sheath of the hair follicle, and K75 is characteristic of the cells forming the companion layer of the inner root sheath of hair follicles. The keratin filaments containing K75 are oriented perpendicularly to the longitudinal axis of the hair like the 'hoops of a barrel'.

### Hair forming compartment keratins

Hair forming compartment keratins are believed to be important for hair fibre integrity and strength and cuticle surface quality. For example, disorders involving cortical keratin genes such as KRT81, 83 and 85 have been shown to lead to instability in keratin filaments and result in hair fibres which are more prone to fracture and weathering.

Accordingly, examples of improved structural properties which may be imparted to the emergent hair fibre in accordance with this invention include improved hair fibre integrity and strength and improved cuticle surface quality (as a consequence of the upregulation of genes encoding hair forming compartment keratins).

Specific examples of hair forming compartment keratins encoded by genes whose expression can be upregulated by piroctone olamine in accordance with this invention are:
Type I (acidic) hair forming compartment keratins, particularly K32, K35 and K36 which are encoded by KRT32, KRT35 and KRT36 respectively. K32 is expressed at the bottom and mid-height of the hair cuticle. K35 is expressed in the bottom of the hair cuticle, and K36 is expressed in the advanced differentiation of the hair cortical cells.
Type II (basic) hair forming compartment keratins, particularly K82, K83 and K85 which are encoded by KRT82, KRT83 and KRT85 respectively. K82 is coexpressed with K32 early in the development of the suprabasal cells of the hair matrix forming the hair cuticle. K83 is expressed in the advanced processes of keratinization in the suprabasal cells forming the hair cortex, and K85 is produced in the keratinizing cuticular cells of the hair. In the hair matrix, K85 forms filaments with K35. In the lower portion of the hair cortex, K85 forms keratin filaments with K31.

### Keratin-associated proteins

Keratin-associated proteins (KAPs) are believed to be important for hair fibre integrity and strength. For example, disorders involving KAP genes have been shown to lead to a reduction in the number of molecules available for KIFs cross-linking, resulting in brittle hair fibres that break easily.

Accordingly, examples of improved structural properties which may be imparted to the emergent hair fibre in accordance with this invention include improved hair fibre integrity and strength (as a consequence of the upregulation of genes encoding KAPs).

Specific examples of KAPs encoded by genes whose expression can be upregulated by piroctone olamine in accordance with this invention are:
KAP10-2, KAP10-7, KAP13-2 and KAP26-1 which are encoded by KRTAP10-2, KRTAP10-7, KRTAP13-2 and KRTAP26-1 respectively. KAP10-2 and KAP10-7 are expressed in a narrow region of the middle and upper keratogeneous zone in the hair fibre cuticle, lying approximately 20 cell layers above the apex of the dermal papilla of the hair root. KAP13-2 is expressed in the middle and upper keratogeneous zone in the hair fibre cuticle and also in the upper matrix and the entire hair cortex. KAP26.1 shows expression which is limited to the hair follicle and localized to the differentiated portion of the hair cuticle.

### Formulations

The piroctone olamine for use according to the invention is suitably formulated into a personal care composition which is suitable for topical application to the hair and scalp.

Suitable personal care compositions for use in the invention include rinse-off or leave-on hair and scalp care compositions such as shampoos, conditioners, creams, lotions, gels, serums, mousses or oils. Rinse-off hair and scalp care compositions such as shampoos and conditioners are preferred.

The piroctone olamine will generally be included in a composition for use in the invention at a level of from 0.05 to 5%, more preferably from 0.1 to 2%, most preferably from 0.2 to 1% (by weight based on the total weight of the composition).

Compositions for use in the invention will generally include a cosmetically acceptable vehicle. The term "cosmetically acceptable" means that the vehicle is suitable for topical application to the skin, has good aesthetic properties, is compatible with the at least one biotin binding compound and the at least one precursor for biotin biosynthesis selected from pimelic acid and/or salts thereof and any other ingredients, and will not cause any safety or toxicity concerns.

The vehicle may comprise an aqueous phase, an oil phase, an alcohol, a silicone phase or a mixture thereof, and may be in the form of an emulsion. Emulsions can have a range of consistencies including thin lotions (which may also be suitable for spray or aerosol delivery), creamy lotions, light creams and heavy creams.

Compositions for use in the invention may also be formulated in a single-phase carrier such as a hydrophobic or hydrophilic liquid. Suitable hydrophobic liquid carriers include liquid polyorganosiloxanes, mineral oils, hydrogenated polyisobutene, polydecene, paraffins and isoparaffins of at least 10 carbon atoms, aliphatic or aromatic ester oils (such as isopropyl myristate, lauryl myristate, isopropyl palmitate, diisopropyl sebacate, diisopropyl adipate and C₁₂ to C₁₅ alkyl benzoates), polyglycol ethers (such as polyglycol butanol ethers) and mixtures thereof. Suitable hydrophilic liquid carriers include water, monohydric or polyhydric aliphatic alcohols having 2 to 8, preferably 2 or 3 carbon atoms (such as ethanol and isopropanol, oligoglycol ethers having 2 to 5 repeat units (such as dipropylene glycol) and mixtures thereof.

Liquid form compositions for use in the invention may be thickened, for example using one or more water soluble or colloidally water soluble polymeric thickening agents. Suitable water soluble or colloidally water soluble polymeric thickening agents include hydroxyethyl cellulose, methyl cellulose, hydroxypropyl methyl cellulose, polyquaternium-10, carrageenan, guar gum, hydroxypropyl guar gum, xanthan gum, polyvinylalcohol, acrylic acid/ethyl acrylate copolymers, carboxyvinyl polymers, cross-linked polyacrylate polymers and polyacrylamide polymers.

Preferred types of composition for use in the invention include shampoos, oils and lotions, which are intended for topical application to the hair and scalp.

Shampoo compositions for use in the invention are generally aqueous (i.e. they have water or an aqueous solution as their major component), and will suitably comprise from 50 to 98%, preferably from 60 to 90% water (by weight based on the total weight of the composition).

Shampoo compositions for use in the invention will typically comprise one or anionic surfactants such as sodium oleyl succinate, ammonium lauryl sulfosuccinate, ammonium lauryl sulphate, sodium dodecylbenzene sulfonate, triethanolamine dodecylbenzene sulfonate, sodium cocoyl isethionate, sodium lauryl isethionate, sodium N-lauryl sarcosinate, sodium lauryl sulfate, sodium lauryl ether sulfate (n) EO, (where n ranges from 1 to 3), ammonium lauryl sulfate and ammonium lauryl ether sulfate (n) EO, (where n ranges from 1 to 3) .

Mixtures of any of the above described materials may also be used.

The total amount of anionic surfactant in shampoo compositions for use in the invention generally ranges from 5 to 30%, preferably from 8 to 20% (by weight based on the total weight of the composition).

Shampoo compositions for use in the invention may also include co-surfactants such as nonionic surfactants, which can be included in an amount ranging from 0.5 to 8%, preferably from 2 to 5% (by weight based on the total weight of the composition) and/or amphoteric or zwitterionic surfactants, which can be included in an amount ranging from 0.5 to 8%, preferably from 1 to 4% (by weight based on the total weight of the composition). Representative nonionic surfactants include alkanolamides such as cocamide monoethanolamide and cocamide monoisopropanolamide; alkyl polyglucosides such as cocoglucoside and lauryl glucoside; and acyl glucamides such as cocoyl methyl glucamide.

Mixtures of any of the above described materials may also be used.

Shampoo compositions for use in the invention may also include one or more cationic polymers, which can be included in an amount ranging from 0.01 to 5%, preferably from 0.05 to 2% (by weight based on the total weight of the composition). Representative cationic polymers include cationic polysaccharide polymers such as cationic cellulose derivatives and cationic guar gum derivatives such as guar hydroxypropyltrimethylammonium chloride.

Shampoo compositions for use in the invention may also include one or more suspending agents, which can be included in an amount ranging from 0.05 to 5%, preferably from 0.1 to 3% (by weight based on the total weight of the composition). Representative suspending agents include polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives such as ethylene glycol distearate.

Hair oils and lotions for use in the invention typically have an oil phase containing at one or more cosmetically acceptable fatty materials which may be liquid or solid at room temperature (25°C). Lotions are typically aqueous emulsions having an aqueous phase in addition to the oil phase.

Suitable cosmetically acceptable fatty materials include naturally derived oils (such as sunflower oil, borage oil, soybean oil, castor oil, olive oil and almond oil); esters of monoalcohols or of polyols with monocarboxylic or polycarboxylic acids, at least one of the alcohols and/or acids comprising at least one hydrocarbon-based chain containing at least 6 carbon atoms (such as octyl palmitate, isopropyl myristate, isopropyl palmitate, isopropyl isostearate, hexyl laurate, isohexyl laurate, isohexyl palmitate, decyl oleate, isodecyl oleate, hexadecyl stearate, decyl stearate, dihexyldecyl adipate, lauryl lactate, myristyl lactate, cetyl lactate, oleyl stearate, oleyl oleate, oleyl myristate, lauryl acetate, cetyl propionate, isononyl isononanoate, propylene glycol dicaprate, diisopropyl adipate, dibutyl adipate, and oleyl adipate); ethers (such as dicapryl ether); fatty alcohols (such as cetyl alcohol, stearyl alcohol and behenyl alcohol); isoparaffins (such as isooctane, isododecane and isohexadecane); silicone oils (such as cyclomethicone, dimethicone and dimethiconol); hydrocarbon oils (such as mineral oil, petrolatum and polyisobutene); fatty acids containing from 8 to 30 carbon atoms, (such as stearic acid, lauric acid, palmitic acid and oleic acid); vegetable fats (such as cocoa butter, coconut oil, palm oil and shea butter); petroleum-based, natural and synthetic waxes (such as lanolin wax, beeswax, carnauba wax, candelilla wax, paraffin wax, lignite wax, microcrystalline waxes, ceresin, ozokerite, and polyethylene waxes); hydrogenated oils which are solid at 25° C (such as hydrogenated castor oil, hydrogenated jojoba oil, hydrogenated palm oil, hydrogenated tallow and hydrogenated coconut oil);and fatty esters that are solid at 25°C (such as C₂₀-₄₀ alkyl stearate).

The aqueous phase of lotions for use in the invention may also include one or more organic liquids that are miscible with water at room temperature (25°C). Exemplary water-miscible organic liquids include monohydric and polyhydric alcohols and derivatives thereof such as C₂-C₆ alkanols (such as ethanol and isopropanol); C₂-C₁₀ glycols and polyols (such as glycerol, propylene glycol, butylene glycol, pentylene glycol, hexylene glycol, caprylyl glycol, dipropylene glycol, and diethylene glycol); C₃-C₁₆ glycol ethers (such as mono-, di-, or tripropylene glycol (C₁-C₄) alkyl ethers and mono-, di-, or triethylene glycol (C₁-C₄) alkyl ethers) and polyethylene glycol having 2 to 12 oxyethylene units.

Lotions for use in the invention may also include surface active ingredients, such as emulsifiers and solubilizers, to enable two or more immiscible components to be combined homogeneously and to help stabilize the composition. Emulsifiers that may be used to form O/W or W/O emulsions include sorbitan oleate, sorbitan sesquioleate, sorbitan isostearate, sorbitan trioleate, PEG-20 sorbitan isostearate, polyglyceryl-3-diisostearate, polyglycerol esters of oleic/isostearic acid, polyglyceryl-6 hexaricinolate, polyglyceryl-4-oleate, polyglyceryl-4 oleate/PEG-8 propylene glycol cocoate, polyglyceryl-2 dipolyhydroxystearate, PEG-30 dipolyhydroxystearate, oleamide DEA, TEA myristate, TEA stearate, magnesium stearate, sodium stearate, potassium laurate, potassium ricinoleate, sodium cocoate, sodium tallowate, potassium castorate, sodium oleate, cetyl phosphate, diethanolamine cetyl phosphate, potassium cetyl phosphate, sodium glyceryl oleate phosphate, dimethicone copolyol, cetyl dimethicone copolyol, octyldimethicone ethoxyglucoside copolyol, dimethicone copolyol crosspolymer and laurylmethicone copolyol.

Combinations of any of the above described materials or product forms may also be used.

Compositions for use in the invention (as described above) may include additional actives for improving the physical and/or aesthetic characteristics of the scalp and/or the hair. Examples include amino acids, vitamins, minerals and/or antioxidants, emollients, humectants, sunscreens, anti-irritants, exfoliating agents, botanical extracts (such as pomegranate, white birch, green tea, chamomile and licorice extracts) and mixtures thereof.

Compositions for use in the invention (as described above) may include additional functional ingredients for improving the physical and/or aesthetic characteristics of the composition per se. Examples include inorganic pigments (such as titanium oxide, zirconium oxide, cerium oxide, zinc oxide, iron oxide, chromium oxide, manganese violet, ultramarine blue, chromium hydrate and ferric blue); organic pigments (such as carbon black and the organic lakes of barium, strontium, calcium or aluminium); pearlescent agents (such as mica coated with titanium oxide and/or iron oxide); dyes, preservatives (such as disodium EDTA, benzyl alcohol, methylparaben, phenoxyethanol, propylparaben, ethylparaben, butylparaben and isobutylparaben); pH adjusters and fragrances (such as essential oils, flower oils, natural extracts from resins, gums, balsams, beans, mosses and other plants, as well as synthetic aromatic materials).

Mixtures of any of the above described materials may also be used.

### Packaging and Use

A composition for use in the invention (as described above) may be packaged in a suitable container to suit its viscosity and intended use by the consumer. For example, a liquid composition can be packaged in a bottle or tube, or in a container fitted with a pump suitable for finger operation, or in a propellant-driven aerosol device. Gel or cream compositions can be packaged in a non-deformable bottle or squeeze container, such as a tube or a lidded jar, or in an applicator having a dispensing head provided with at least one aperture through which the composition can be extruded under mild pressure.

The composition is suitably applied to the hair and scalp and massaged into the surface of the scalp. Generally, an amount corresponding to about 1 to 15 ml of the composition per application is applied uniformly over the area of treatment daily or at least once a week over a time interval of at least 7 (seven) days, more preferably at least 30 (thirty) days.

The invention will be further illustrated by the following, non-limiting Examples.

### EXAMPLES

### Method

Sixty female subjects were recruited for the study and classified as healthy (30 subjects) or dandruff sufferers (30 subjects) based on total weighted head score assessment. 4mm scalp biopsy samples from both subject groups were taken at two different timepoints: at baseline, and after 4 weeks of treatment with a shampoo containing 0.5 wt% piroctone olamine. Half of each biopsy was homogenised and subjected to RNA extraction, QuantSeq3'FWD library preparation (Lexogen), and RNA sequencing on NextSeq500 platform (Illumina). Raw and normalised RNA-seq data were subject to rigorous QC evaluation, all samples passed QC test and were included in subsequent analysis.

### Results

Statistical models were fitted to the data to identify differentially expressed genes in the dandruff group compared to the healthy group.

In the scalp biopsy samples taken at baseline, the specific genes shown in Table 1 were found to be significantly downregulated in the dandruff group, as compared to the healthy group.

**Table 1**

| Gene symbol | Protein | Protein localisation | Dandruff vs healthy at baseline | |
|---|---|---|---|---|
| | | | Fold Change | P value |
| *Keratins of Inner Root Sheath^{†}* | | | | |
| KRT25 | K25 | IRS: Icu, Hu, He | -1.56 | 0.0028 |
| KRT26 | K26 | IRS: Icu | -1.43 | 0.0216 |
| KRT27 | K27 | IRS: Icu, Hu, He | -1.55 | 0.0036 |
| KRT28 | K28 | IRS: Icu, Hu, He | -1.48 | 0.0075 |
| KRT71 | K71 | IRS: Icu, Hu, He | -1.56 | 0.0029 |
| KRT72 | K72 | IRS: Icu | -1.48 | 0.0137 |
| KRT73 | K73 | IRS: Icu | -1.54 | 0.0076 |
| KRT74 | K74 | IRS: Hu | -1.53 | 0.0083 |

| *Keratins of Hair Forming Compartments* | | | | |
|---|---|---|---|---|
| KRT32 | K32 | Cuticle | -1.60 | 0.0044 |
| KRT35 | K35 | Cortex, Cuticle | -1.66 | 0.0062 |
| KRT36 | K36 | Cortex | -1.47 | 0.0249 |
| KRT75 | K75 | Medulla, Companion layer | -1.43 | 0.0058 |
| KRT82 | K82 | Cuticle | -1.51 | 0.0233 |
| KRT83 | K83 | Cortex | -1.63 | 0.0283 |
| KRT85 | K85 | Cortex, Cuticle | -1.57 | 0.0151 |

| *Keratin Associated Proteins* | | | | |
|---|---|---|---|---|
| KRTAP10-2 | KAP10-2 | Cuticle | -1.57 | 0.0216 |
| KRTAP10-7 | KAP10-7 | Cuticle | -1.41 | 0.0374 |
| KRTAP13-2 | KAP13-2 | Cortex, Cuticle | -1.74 | 0.0220 |
| KRTAP26-1 | KAP26-1 | Cuticle | -1.44 | 0.0445 |

| | | | | |
|---|---|---|---|---|
| *^{†} localisation within Inner Root Sheath (IRS) refers to inner root sheath cuticle (Icu), Huxley layer (Hu), Henle layer (He).* | | | | |

As shown in Table 2, after 4 weeks of treatment with 0.5wt% piroctone olamine shampoo the same genes showed a trend towards upregulation, indicative of a shift towards healthy state.

**Table 2**

| Gene symbol | Protein | Protein localisation | Dandruff vs healthy after treatment | |
|---|---|---|---|---|
| | | | Fold Change | P value |
| *Keratins of Inner Root Sheath** | | | | |
| KRT25 | K25 | IRS: Icu, Hu, He | 1.31 | 0.0789* |
| KRT26 | K26 | IRS: Icu | 1.23 | 0.2370* |
| KRT27 | K27 | IRS: Icu, Hu, He | 1.36 | 0.0428 |
| KRT28 | K28 | IRS: Icu, Hu, He | 1.31 | 0.0917* |
| KRT71 | K71 | IRS: Icu, Hu, He | 1.31 | 0.0663* |
| KRT72 | K72 | IRS: Icu | 1.15 | 0.4070* |
| KRT73 | K73 | IRS: Icu | 1.27 | 0.1594* |
| KRT74 | K74 | IRS: Hu | 1.28 | 0.1162* |

| *Keratins of Hair Forming Compartments* | | | | |
|---|---|---|---|---|
| KRT32 | K32 | Cuticle | 1.29 | 0.1179* |
| KRT35 | K35 | Cortex, Cuticle | 1.44 | 0.0542* |
| KRT36 | K36 | Cortex | 1.31 | 0.1886* |
| KRT75 | K75 | Medulla, Companion layer | 1.29 | 0.0435 |
| KRT82 | K82 | Cuticle | 1.29 | 0.1776* |
| KRT83 | K83 | Cortex | 1.40 | 0.1001* |
| KRT85 | K85 | Cortex, Cuticle | 1.39 | 0.0671* |

| *Keratin Associated Proteins* | | | | |
|---|---|---|---|---|
| KRTAP10-2 | KAP10-2 | Cuticle | 1.38 | 0.1021* |
| KRTAP10-7 | KAP10-7 | Cuticle | 1.36 | 0.0850* |
| KRTAP13-2 | KAP13-2 | Cortex, Cuticle | 1.95 | 0.0005 |
| KRTAP26-1 | KAP26-1 | Cuticle | 1.34 | 0.1411* |
| * Not significant | | | | |

### Conclusions

The results in Table 1 show that the gene expression of multiple hair specific keratins and keratin-associated proteins is negatively affected by the condition of dandruff. Such decrease in expression of vital structural components of the hair shaft, may perturb deposition of keratins and KAPs during pre-emergent hair differentiation and fibre formation and in turn adversely affect emergent fibre properties.

However, Table 2 shows that the treatment with piroctone olamine in accordance with the invention restores the keratin and KAP gene expression profile towards one that resembles healthy signature.

This indicates that treatment with piroctone olamine can improve hair fibre properties by reversing negative changes in keratin and KAP gene expression, resulting in thicker, stronger and healthier looking hair.

## Claims

1. Cosmetic use of piroctone olamine as an active ingredient for upregulating the expression of genes encoding hair fibre structural proteins in cells of the pre-emergent hair fibre; and thereby improving the structural properties of the emergent hair fibre.

2. Cosmetic use according to claim 1, in which the piroctone olamine is formulated into a rinse-off hair and scalp care composition.

3. Cosmetic use according to claim 1 or claim 2, in which the piroctone olamine is included in the rinse-off hair and scalp care composition at a level of from 0.05 to 5% (by weight based on the total weight of the composition).

4. Cosmetic use according to any one of claims 1 to 3, in which the genes encoding hair fibre structural proteins are selected from genes encoding inner root sheath (IRS) keratins.

5. Cosmetic use according to claim 4, in which the genes encoding IRS keratins are selected from any or all of: type I (acidic) inner root sheath keratin genes KRT25-28 and type II (basic) inner root sheath keratin genes KRT71-75.

6. Cosmetic use according to claim 4 or claim 5, in which the improved structural properties imparted to the emergent hair fibre are improved hair fibre texture and alignment.

7. Cosmetic use according to any one of claims 1 to 3, in which the genes encoding hair fibre structural proteins are selected from genes encoding hair forming compartment keratins.

8. Cosmetic use according to claim 7, in which the genes encoding hair forming compartment keratins are selected from any or all of: type I (acidic) hair forming compartment keratin genes KRT32, KRT35 and KRT36 and type II (basic) hair forming compartment keratin genes KRT82, KRT83 and KRT85.

9. Cosmetic use according to claim 7 or claim 8, in which the improved structural properties imparted to the emergent hair fibre are improved hair fibre integrity and strength and improved cuticle surface quality.

10. Cosmetic use according to any one of claims 1 to 3, in which the genes encoding hair fibre structural proteins are selected from genes encoding keratin-associated proteins (KAPs).

11. Cosmetic use according to claim 10, in which the genes encoding KAPs are selected from any or all of: KRTAP10-2, KRTAP10-7, KRTAP13-2 and KRTAP26-1.

12. Cosmetic use according to claim 10 or claim 11, in which the improved structural properties imparted to the emergent hair fibre are improved hair fibre integrity and strength.
